# EUROPEAN PATENT SPECIFICATION

(11) **EP 3 078 355 B1**
(45) Date of publication and mention of the grant of the patent: **05.12.2018**
(21) Application number: 14867169.6
(22) Date of filing: 16.10.2014
(51) Int. Cl.: A61F 5/03, A61F 5/02

(54) **STRUCTURAL ARRANGEMENT FOR A DYNAMIC THORAX COMPRESSOR**
STRUKTURELLE ANORDNUNG FÜR EINEN DYNAMISCHEN THORAX-VERDICHTER
AGENCEMENT STRUCTURAL POUR COMPRESSEUR DYNAMIQUE DE THORAX

(30) Priority: 06.12.2013 BR PI1331509
(43) Date of publication of application: 12.10.2016
(73) Proprietor: Haje, Davi De Podesta, CEP 70710-100 Asa Norte, DF (BR)
(72) Inventor: Haje, Davi De Podesta, CEP 70710-100 Asa Norte, DF (BR)
(74) Representative: Habermann, Hruschka & Schnabel
(86) International application number: PCT/BR2014/000379
(87) International publication number: WO 2015/081397

(56) References cited:
- WO-A2-2009/028836
- AR-A1- 041 766
- BR-U- 8 203 124
- CN-Y- 2 158 694
- FR-A1- 2 863 870
- KR-A- 20100 051 249
- US-A1- 2011 028 875
- US-A1- 2012 130 371

## Description

The present invention relates to a constructive disposition inserted in a dynamic thorax compressor, and more specifically, to laterolateral and anteroposterior regulation means of the dynamic thorax compressor, as well as in the coupling of the plates where the front pads are fixed in different positions, without need for a rivet.

Deformities in the anterior thoracic-wall deformities, universally designated as "pectus" deformities, are frequently observed in the medical practice. Studies report one case of "pectus excavatum", known as "excavated thorax", for each 300 live births, and according to Haje, a case of "pectus" for each 100 schoolchildren examined; however, in face of generated psychological problems, these deformities are usually hidden by their bearers, thus making them unknown. The misfortune of "pectus" deformity influences all areas of the patient's life, and, according to studies performed, the psychological effects are higher after 11 years old, due to feelings of embarrassment, social anxiety, shame, limited capacity for activities and communication, negativism, intolerance, frustration and even depressive reactions, however, adequate medical support to the bearer of a "pectus" deformity can contribute to the restoration of the mental health of the patient, and its reintegration into normal social interaction, bringing relief to him and to his family.

Haje AS et al. discovered in humans, and through experiments in animals, that the formation and growth of the anterior thoracic-wall is endochondral, with the presence of cartilaginous growth plates between the segments of the growing sternum and in the costochondral joints. The formation of the sternum occurs from two longitudinal bands of the mesoderm, from the shoulder region, which join in the craniocaudal direction, as it develops a condrification process. The costal cartilages, originating from the vertebral column, merge to the sternum in formation, and cartilage growth plates develop in these gathering places of the costal cartilages with the sternum, and are responsible for sternal growth. Haje SA et al. further demonstrated that the coastal growth plates are also present in the costochondral junction of the costal arches. When dealing with long bones of children and teenagers, the orthopedic surgeon avoids harm cartilage growth plates, since such injury will cause deformity in the future. However, this care does not seem to exist in relation to the thorax, given that, among dozens of surgical techniques described to treat the "pectus" deformities, many do not draw attention to sternal and costal growth plates.

Growth disorders of bone and cartilage of the anterior thoracic-wall are described in specialized literature as etiological agents; thus, "pectus" deformities tend to the natural progression in the growth peak of adolescence. Biomechanical factors, such as respiratory disorders, scoliosis and kyphosis, may contribute to the pathogenesis of "pectus" deformities.

Haje still published several radiographic unprecedented studies about the "pectus".

From 1977 to November 2013, Haje SA and Haje DP examined 5,150 patients bearing "pectus" deformities, being 73% of males and 27% of females. Haje SA and Haje DP proposed a classification according to the anatomical location of the protruding or depressed area. A predominantly protruding deformity in the sternal region, chondrosternal junction or costal cartilage adjacent to the sternum was classified as "pectus carinatum", known as pigeon breast, while the exclusive presence of sternal depression was classified as "pectus excavatum". "Pectus carinatum" was classified according to the anatomical location of the apex of the protrusion, in: lower "pectus carinatum" (PCI); side "pectus carinatum" (PCL), and higher "pectus carinatum" (PCS). "Pectus excavatum" was classified, according to the extent of the depression, in the types localized (PEL) and broad (PEA). Additionally to the basic types of "pectus", "mixed" types of deformities were also detected, the diagnosis being always based on the predominant deformity, with the secondary deformity/deformities being noted in addition to the main diagnosis, such as, for example: PCS + PEL + left PCL; right PCL + PEL; PEA + left PCL, PCI + right PCL, etc. Protrusions of the costal edges at the lower part of the anterior thoracic-wall, when present, have been also recorded for appropriate follow-up and therapeutic characterization. In the cases of Haje DP and Haje AS, "pectus carinatum" proved to be more frequent.

Haje DP and Haje SA investigated, in each patient, the occurrence of any concomitant skeletal disease and previous surgical procedure on the sternum, and they detected three forms of occurrence: the pathological, the iatrogenic and idiopathic. The pathological, that occurs in the presence of diseases associated with general growth disorders, such as Marfan syndrome, bone dysplasia and osteogenesis imperfecta, was also diagnosed.

Patients and/or guardians were asked about the presence of other cases in the family (heredity) and biomechanical factors that could influence the growth and development of the anterior thoracic-wall, such as respiratory disorders (asthma, pneumonia, adenoid hypertrophy, sinusitis and allergic rhinitis). Physical and, when necessary, radiological examinations, were routinely made to search for column deviations (exacerbated thoracic kyphosis).

The treatment with dynamic compression orthosis, known as dynamic thorax compressor I - CDT I was initially described by Haje in 1979 for treating "pectus carinatum". The CDT I system initially described by Haje had shoulder straps and screws on the sides, with the possibility of gradual compression on protrused areas of the thorax. The straps were later eliminated from CDT by Haje. After the initial description of the CDT I of Haje, compressive orthesis described by third parties with velcro or snaps on the sides were adopted as conservative therapeutic options, but the use of velcro was not effective for children with flexible types of "pectus carinatum". The treatment with CDT orthosis, with screws on the sides, was firstly described by Haje in the literature, and from 1988 also for the treatment of "pectus excavatum ', when CDT II was created, which has the same compressive mechanism of CDT I, but has the plates with pads to support the thorax adapted for compression on protrusion areas of coastal edges. In 2006, the current applicant synthesized the description of his method, with the publication of the term Dynamic Remodeling (DR) method, or DR method, to designate the use of CDT orthosis, simultaneously to the practice of exercises to promote an increase of intrathoracic pressure. The method involves the balance of forces on the thorax, while the orthosis applies outer dynamic pressure on the protruding or salient areas during exercises, simultaneously with the use of one or two CDT orthosis (I and/or II), thus promoting inner pressure on depressed areas and the remodeling of the ribcage as a whole.

According to Haje, the corrective remodeling of "pectus excavatum", therefore, only happens when a compression of the costal edges potentiates the increased of intrathoracic pressure caused by exercises.

The option of treating conservatively, by the method of Haje, the "pectus" deformities, is based on the principles of Nicolas Andry, considered "the father of Orthopedics", and the effects of this treatment can be explained by the law of bone remodeling by Julius Wolff. According to Haje theory, therapeutic forces regularly applied on deformed cartilage and bones can produce a gradual, beneficial and corrective remodeling, and this can be particularly observed in the anterior thoracic-wall, a malleable region.

In the cases of the patients treated by Haje, the prescription of orthosis for adults happened only to patients extremely uncomfortable with the aesthetic aspect of the thorax, and it was always preceded with the explanation about the difficulties of the treatment in adulthood. For growing individuals, the following criterion was adopted: in childhood, for PCS bearers (a rigid type of deformity since childhood); in pre-adolescence, for PEL and PEA bearers; and in adolescence, for patients with PCI and PCL (more flexible types of deformities). In childhood, PCI, PCL, PEL and PEA bearers just received prescription of orthosis in the case of a frequent respiratory disorder history or, eventually, in case of mild to severe or progressive deformity. The orthosis was not prescribed to discrete deformities to very young children, with PCI, PCL, PEL and PEA, without history of respiratory disorder; in such cases, only photographic documentation and follow-up were indicated. All the above criterion of indication of treatment was devised by Haje SA and Haje DP.

Clinic images of the thorax of each patient were routinely made, always form the same side oblique angle, for follow-up. The evaluation of the treatment results has been classified on the basis of the analysis of the images and of improvement indexes (IM), where 3 corresponds to a good or excellent improvement, 2 corresponds to a moderate improvement, 1 corresponds to a discrete improvement and 0 corresponds to no improvement. This criterion of treatment evaluation was also designed by Haje.

In the Haje cases, the minimal follow-up time of one month has been adopted, given that, during this time, an improvement or even the hypercorrection for flexible "pectus carinatum" can be observed, but this does not mean that the treatment should be interrupted. The shorter treatment time, the greater the likelihood of recurrence and the medical follow-up is critical to the proper remodeling of the anterior thoracic-wall. If protrusions costal edges are formed, or if they exacerbate with the compression of a sternal protrusion, the CDT II orthosis must be added to the treatment. The removal of orthosis should be gradually carried out, and the medical discharge should only happen when the correction stabilization is assured.

The hypercorrection of the original deformity was detected in 61 cases, and was fixed in all cases, by means of individual practices.

In cases where CDT orthosis was/were used four hours daily, with a DR method exercise program that included stretching the column vertically and side pushups, in greater number of times on the side which featured improvement of the curve in side slopes of pre-treatment radiographs, and the use of CIB for the rest of the day, the patients with "pectus" deformities and scoliosis of angular value between 20° and 52° which received, besides of the treatment by DR method, a concomitant treatment with Brasilia inclined vest or CIB, 8 showed improvement of at least 5° on the scoliotic curve, and in one of the cases, the improvement was 20°.

According to Haje, the DR method requires criteria and appropriate medical supervision, which must be extended, since it may take one or more years for the correction to stabilize, depending on how the treatment is run by the patient over time. Treatment with CDT I and/or CDT II orthoses must be started according to the age and type of deformity. The orthosis must be constructed individually, according to the plaster cast, with formats and measurements of the pads marked on the mold, in accordance with the medical specifications prescribed. The front and rear rods must be of aluminium, to allow adjustments as the treatment progresses, since the patient grows and/or the shape of its thorax is modified by the treatment. The side rods must be made from small tubes with nuts on its front edge, and the screws that are threaded into these tubes should always be used as a compression mechanism, for the gradual adjustment, to the extent that the osteocartilaginous structures deflects, thus bringing dynamism to the treatment. The exercises complement the dynamics of the treatment, by movements of the ribcage and the promotion of inner pressure in depressed areas of the anterior thoracic-wall. The ideal compression by tightening the screws, until firming the orthosis on the patient's thorax, should be determined by the doctor in the consultation for placing the same, and the progress of tightening and compression degree should happen according to the tolerance of the patient. One of the frequently necessary adjustments, as the patient grows and the thorax changes with the treatment, is the laterolateral enlargement. Such enlargement occurs by opening the angles of the rear rod and of the front rod in a prosthetic workshop using specialized instrumental. The decrease in the length of the screws and of the side rods or advance of the bending angle of the front rod may be required to provide additional compression. The curving of the middle part of the front and rear rods often occurs, and thus, the doctor needs to prescribe rectification of these pieces, in addition to duplication for reinforcement, so that the compression becomes efficient again. Such modifications and adjustments provide possibility of additional tightening the screws that had completely penetrated in the side rods. The change in position, size and shape of the pads may be needed as well. All the adjustments above have been described in the literature, by Haje.

From BR 8 203 124 U, which is regarded as the closest prior art, a dynamic thorax compressor is known. Said dynamic thorax compressor comprises two front and rear transverse rod segments, two side rods, and two front and rear compression pads fixed to the transverse rod segments. The dynamic thorax compressor further includes a transverse compressor regulation means comprising spindles trespassing openings at outer ends of the front transverse rod segment, wherein the spindles are threaded into the front ends of the side rods through knobs.

Further systems are disclosed in CN 2158694 Y, KR 2010-0051249 A and FR 2,863,870 A1.

Hence, it is necessary to propose appropriate means for laterolateral and anteroposterior adjustments of the orthosis, and the coupling of the front pad plates in different positions, without needing a rivet. All these modifications allow better monitoring of the gradual evolution of the treatment, which is the main objective of this patent. The orthosis formerly named CDT I and II, is called now CDTA I and II, with "A" meaning adjustable.

The present invention provides a dynamic thorax compressor having the features of claim 1.

In order to better understand the present invention, reference is made to the drawings attached, wherein:
Figure 1 - illustrates the perspective view of the dynamic thorax compressor CDTA1, for the treatment of "pectus carinatum", incorporating the constructive disposition currently proposed;
Figure 2 - illustrates the exploded perspective view of the dynamic thorax compressor CDTA1, for the treatment of "pectus carinatum", incorporating the constructive disposition currently proposed;
Figure 3 - illustrates the top view of the dynamic thorax compressor CDTA1, for the treatment of "pectus carinatum", incorporating the constructive disposition currently proposed;
Figure 4 - illustrates the side view of the dynamic thorax compressor CDTA1, for the treatment of "pectus carinatum", incorporating the constructive disposition currently proposed;
Figures 5 and 6 - illustrate rear views of the dynamic thorax compressor CDTA1, for the treatment of "pectus carinatum", highlighting the laterolateral and anteroposterior adjustments of the dynamic thorax compressor CDTA1;
Figure 7 - illustrates the perspective view of the dynamic thorax compressor CDTA2, for the treatment of "pectus excavatum", incorporating the constructive disposition currently proposed;
Figure 8 - illustrates the exploded perspective view of the dynamic thorax compressor CDTA2, for the treatment of "pectus excavatum", incorporating the constructive disposition currently proposed;
Figure 9 - illustrates the top view of the dynamic thorax compressor CDTA2, for the treatment of "pectus excavatum", incorporating the constructive disposition currently proposed; Figure 10 - illustrates the side view of the dynamic thorax compressor CDTA2, for the treatment of "pectus excavatum", incorporating the constructive disposition currently proposed;
Figures 11 and 12 - illustrate rear views of the dynamic thorax compressor CDTA2, for the treatment of "pectus excavatum", highlighting the laterolateral and anteroposterior adjustments of the dynamic thorax compressor CDTA2.

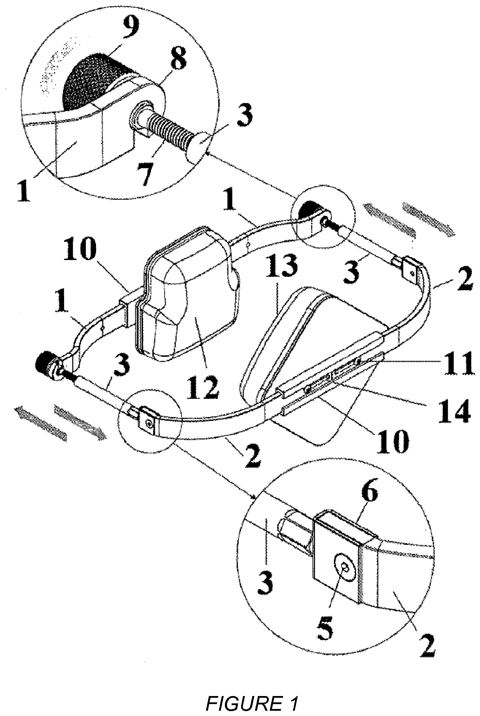

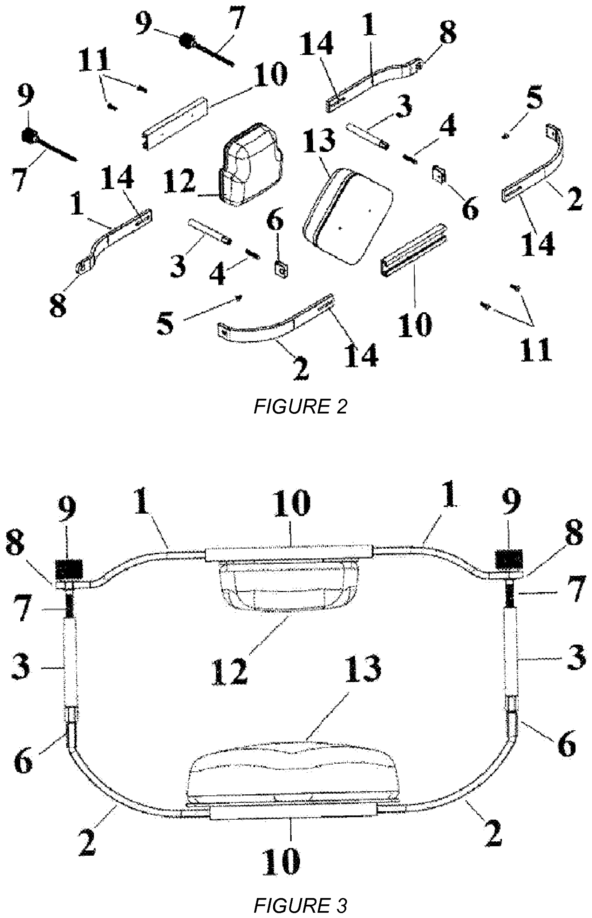

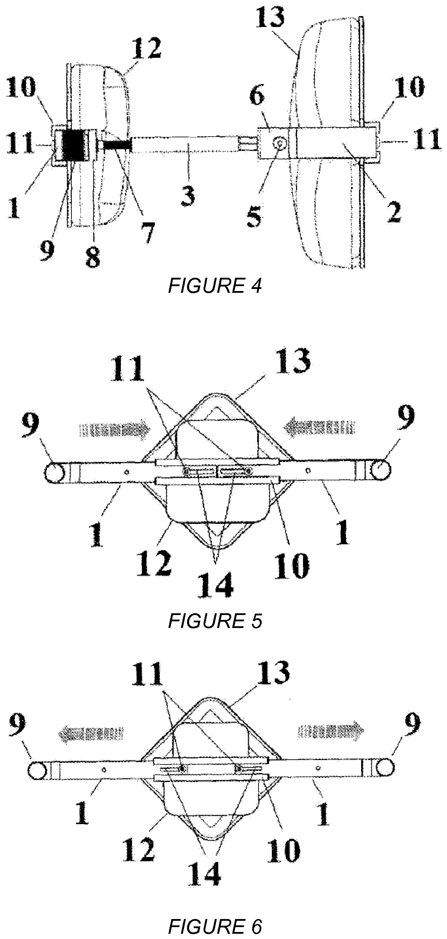

As it is illustrated in figures 1 to 6, the dynamic thorax compressor CDTA1, for use in the treatment of "pectus carinatum", is formed by a structure comprised by front (1) and rear (2) transverse rods, and by side rods (3), in whose rear ends threaded pins (4) are provided that fit into the holes provided on the front edges of the outer ends of said rear rods, and which are retained by screws (5) transversely arranged near the front edges of the outer ends of the rear rods, and where protection caps (6) are provided, as well as spindles (7) which trespass openings (8) provided at the outer ends of said front transverse rods, which are threaded into the front ends of the side rods, through knobs (9) for transverse adjustment of the compressor, and the inner ends of the segments of front and rear transverse rods fitted in rails (10), in which the front (12) and rear (13) compression pads are fixed using screws, wherein the laterolateral compressor regulation is achieved by positioning the elongated slots (14), provided in the inner ends of the front and rear transverse rods in relation to said fastening screws. We emphasize that the side rods (3), as well as front rods (1) and rear rods (2) can have different sizes, as well as rails (10) where the front rods and the rear rods fit. The plates with front (12) and rear (13) pads may have different sizes and arrangements, according to the deformity shape of the patient. These plates with front pads (12) may have threaded holes in different positions on the same, allowing changes in their positioning in relation to the slot in the front rod (1). The transverse or anteroposterior compression is made by tightening the spindles/knobs that enter into the side rod. Another way to increase or decrease the transverse or anteroposterior compression is by changing the side rods (3).

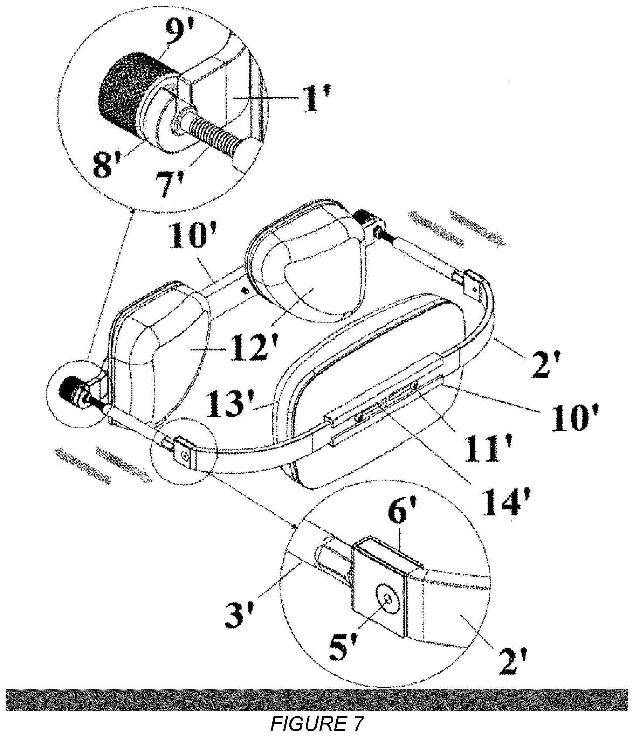

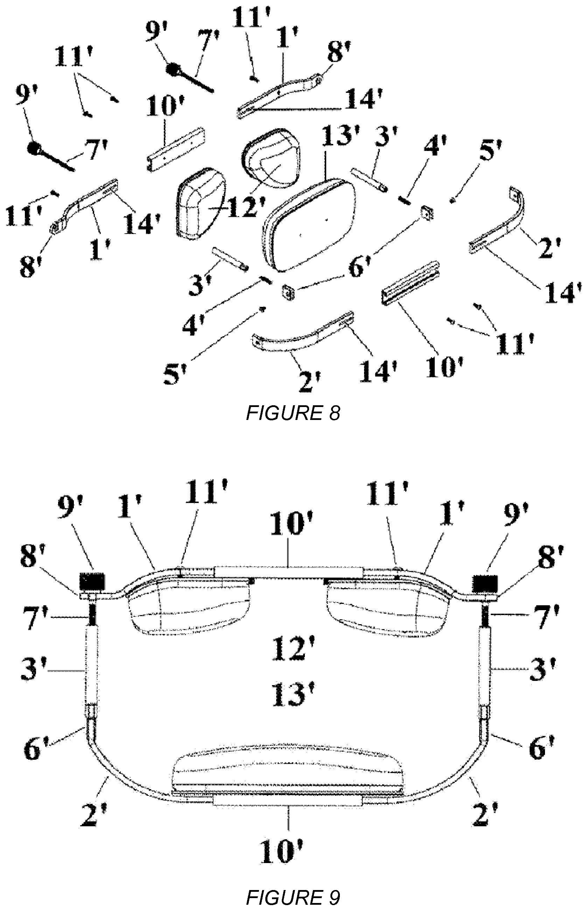

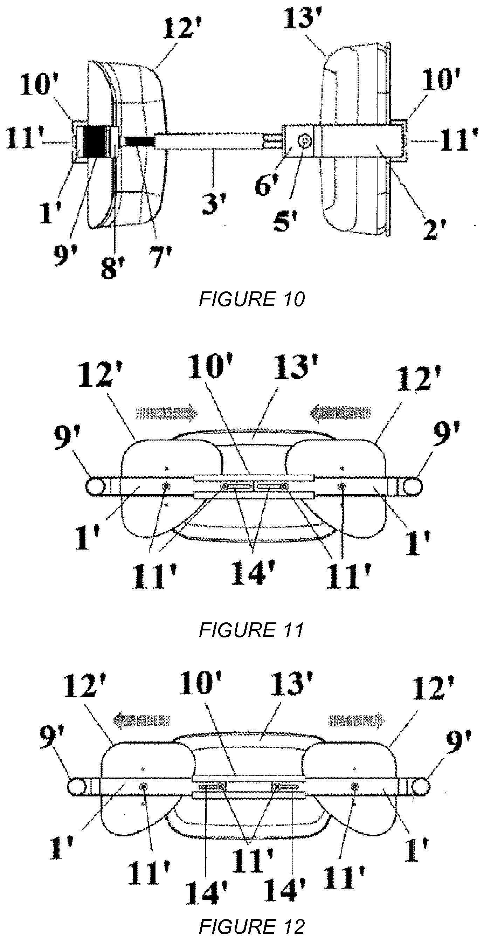

As it is illustrated in figures 7 to 12, the dynamic thorax compressor CDTA2, for use in the treatment of "pectus excavatum", is formed by a structure comprised by front (1') and rear (2') transverse rods, and by side rods (3'), in whose rear ends threaded pins (4') are provided that fit into the holes provided on the front edges of the outer ends of said rear rods, and which are retained by screws (5') transversely arranged near the front edges of the outer ends of said rear rods, and where protection caps (6') are provided, as well as spindles (7') which trespass openings (8') provided at the outer ends of said front transverse rods, which are threaded into the front ends of the side rods, through knobs (9) for transverse adjustment of the compressor, and the inner ends of the segments of front and rear transverse rods are fitted in rails (10), in which the front (12) and rear (13) compression pads are fixed using screws, wherein the laterolateral compressor regulation is achieved by positioning the elongated slots (14), provided in the inner ends of the front and rear transverse rods in relation to said fastening screws. We emphasize that the side rods (3'), as well as front rods (1') and rear rods (2'), can have different sizes, as well as rails (10') where the front rods and the rear rods fit. The plates with front (12') and rear (13') pads may have different sizes and arrangements, according to the deformity shape of the patient. These plates with front pads (12') may have threaded holes in different positions on the same, allowing changes in their positioning in relation to the slot in the front rod (1'). The transverse or anteroposterior compression is made by tightening the spindles/knobs that enter into the side rod. Another way to increase or decrease the transverse or anteroposterior compression is by changing the side rods (3').

## Claims

1. A dynamic thorax compressor comprising:
a) front (1, 1') and rear (2, 2') pairs of transverse rod segments, each transverse rod segment having an inner end and an outer end;
b) side rods (3, 3'), each side rod comprising a front end, a rear end, and a pin (4, 4') in the rear end;
c) a transverse compressor regulation means;
d) a laterolateral compressor regulation means; and
e) front (12, 12') and rear (13, 13') compression pads; wherein the pins (4, 4') of the side rods fit into holes provided in the front edges of the outer ends of the rear transverse rod segments, the pins having protection caps (6, 6'), and the pins being retained by screws (5, 5') transversely arranged near the front edges of the outer ends of the rear transverse rod segments; wherein said compression pads are fixed by means of fastening screws (11, 11') received in rails (10, 10') fitted to the inner ends of the front and rear transverse rods segments; wherein the transverse compressor regulation means comprises spindles (7, 7') trespassing openings (8, 8') provided at the outer ends of the front transverse rod segments, wherein the spindles are threaded into the front ends of the side rods, through knobs (9, 9'); and wherein the laterolateral compressor regulation means comprises elongated slots (14, 14'), provided in the inner ends of the front and rear transverse rod segments, through which the fastening screws are fitted.

2. The dynamic thorax compressor of claim 1, comprising front (12, 12') and rear (13, 13') compression pads in different sizes and arrangements so as to adapt to the deformity to be treated.

## Patentansprüche

1. Dynamischer Thoraxverdichter, aufweisend:
a) vordere (1,1') und hintere (2,2') Paare von Querstabsegmenten, wobei jedes Querstabsegment ein inneres Ende und ein äußeres Ende aufweist;
b) Seitenstäbe (3,3'), wobei jeder Seitenstab ein vorderes Ende, ein hinteres Ende und einen Stift (4,4') in dem hinteren Ende aufweist;
c) ein Querkompressionsregelungsmittel;
d) ein latero-laterales Kompressionsregelungsmittel; und
e) vordere (12,12') und hintere (13,13') Kompressionspolster;
wobei die Stifte (4,4') der Seitenstäbe in Löcher passen, die in den Vorderkanten der äußeren Enden der hinteren Querstabsegmente vorgesehen sind, wobei die Stifte Schutzkappen (6,6') aufweisen und die Stifte durch Schrauben (5,5') gehalten werden, die in der Nähe der Vorderkanten der äußeren Enden der hinteren Querstabsegmente quer angeordnet sind;
wobei die Kompressionspolster mittels Befestigungsschrauben (11,11') befestigt sind, die in Schienen (10,10') aufgenommen sind, die an den inneren Enden der vorderen und hinteren Querstabsegmente angebracht sind;
wobei das Querkompressionsregelungsmittel Spindeln (7,7') aufweist, die Öffnungen (8,8') durchqueren, die an den äußeren Enden der vorderen Querstabsegmente vorgesehen sind, wobei die Spindeln in die vorderen Enden der Seitenstäbe durch Knöpfe (9,9') eingeschraubt sind; und wobei das latero-laterale Kompressionsregelungsmittel längliche Schlitze (14,14') aufweist, die an den inneren Enden der vorderen und hinteren Querstabsegmente vorgesehen sind, durch die die Befestigungsschrauben gesteckt werden.

2. Dynamischer Thoraxverdichter nach Anspruch 1, bei dem die vorderen (12,12') und hinteren (13,13') Kompressionspolster unterschiedliche Größen und Anordnungen aufweisen, um sich an die zu behandelnde Verformung anzupassen.

## Revendications

1. Compresseur thoracique dynamique comprenant :
a) des paires avant (1, 1') et arrière (2, 2') de segments de tige transversale, chaque segment de tige transversale ayant une extrémité intérieure et une extrémité extérieure ;
b) des tiges latérales (3, 3'), chaque tige latérale comprenant une extrémité avant, une extrémité arrière, et une broche (4, 4') dans l'extrémité arrière ;
c) des moyens de régulation transversale de compresseur ; et
d) des moyens de régulation latéro-latérale de compresseur ; et
e) des coussins de compression avant (12, 12') et arrière (13, 13') ;
dans lequel les broches (4, 4') des tiges latérales s'emboîtent dans des trous agencés dans les bords avant des extrémités extérieures des segments de tige transversale arrière, les broches ayant des capuchons de protection (6, 6'), et les broches étant retenues par des vis (5, 5') disposées transversalement à proximité des bords avant des extrémités extérieures des segments de tige transversale arrière ;
dans lequel lesdits coussins de compression sont fixés au moyen de vis de fixation (11, 11') reçues dans des rails (10, 10') montés sur les extrémités intérieures des segments de tiges transversales avant et arrière ;
dans lequel les moyens de régulation transversale de compresseur comprennent des mandrins (7, 7') s'introduisant dans des ouvertures (8, 8') agencées au niveau des extrémités extérieures des segments de tige transversale avant, les mandrins étant filetés dans les extrémités avant des tiges latérales, à travers des boutons (9, 9') ; et
dans lequel les moyens de régulation latéro-latérale de compresseur comprennent des fentes allongées (14, 14'), agencées dans les extrémités intérieures des segments de tige transversale avant et arrière, à travers lesquelles les vis de fixation sont montées.

2. Compresseur thoracique dynamique selon la revendication 1, comprenant des coussins de compression avant (12, 12') et arrière (13, 13') de tailles et dispositions différentes pour s'adapter à la déformation à traiter.
